# EUROPEAN PATENT APPLICATION

(11) **EP 4 379 048 A1**
(43) Date of publication of application: **05.06.2024**
(21) Application number: 22383172.8
(22) Date of filing: 02.12.2022
(51) Int. Cl.: C12N 9/02, C12P 5/02, C12P 7/18, C12P 7/26, C12P 7/44

(54) **METHOD FOR BIODEGRADING POLYOLEFIN-DERIVED POLYMERS**

(71) Applicant: Consejo Superior de Investigaciones Científicas (CSIC), 28006 Madrid (ES)
(72) Inventor: Bertocchini, Federica, 28040 Madrid (ES); Solá i Vilarrubias, María Gracia, 08028 Barcelona (ES); ARIAS PALOMO, Ernesto, 28040 Madrid (ES)
(74) Representative: Pons

(57) **Abstract**

The invention refers to the use of an enzyme from the wax worm (*Galleria mellonella* larvae) saliva which has the unexpected capacity of oxidizing and depolymerizing untreated polyolefin-derived polymers, such as polyethylene (PE), at room temperature (RT), neutral pH and short incubation times. The invention also refers to a method for biodegrading a polyolefin-derived polymer wherein this enzyme is used.

## Description

The invention falls within the field of biotechnology, in particular within plastics, preferably polyolefin-derived plastics, more preferably polyethylene (PE), enzyme biodegradation.

### BACKGROUND ART

Polyethylene (PE) accounts for 30% of synthetic plastic production, largely contributing to plastic waste pollution on the planet to-date. Together with polypropylene (PP), polystyrene (PS) and polyvinylchloride (PVC), PE is one of the most resistant polymers, with very long C-C chains organized in a crystalline, dense structure. Given the hundreds of million tons of plastic waste accumulating and the still escalating pace of plastic production, re-utilization of plastic residues is a necessary path to alleviate the gravity of the plastic pollution problem, and at the same time to render available a huge potential reservoir of carbon. To-date, only mechanical recycling is being applied at a large scale. Several factors, such as the low number of plastic types prone to be mechanically recycled and the low quality of the secondary products, severely restrict the potential of this solution to the problem of plastic waste accumulation. Chemical recycling, as an alternative procedure, is preferentially aiming at plastic upcycling, e.g. decomposing polyolefin-derived plastics in order to take advantage of smaller intermediates. Several technologies have been applied at a lab scale, although the high energetic cost might still impede the scaling up of these technological tools.

In addition to mechanical and chemical recycling, biodegradation is widely considered as a promising strategy to dispose of plastic residues. Biodegradation refers to environmental degradation by biological agents. The IUPAC defines biodegradation as the "breakdown of a substance catalyzed by enzymes *in vitro* or *in vivo",* later modified "to exclude abiotic enzymatic processes". In the case of PE, biodegradation requires the introduction of oxygen into the polymeric chain; this causes the formation of carbonyl groups and the subsequent scission of the long hydrocarbon chains with production of smaller molecules, which can then be metabolized by microorganisms (Albertsson, A.C., Andersson, S. O. and Karlsson, S. (1987). Polymer Degradation and Stability 18, 73-87; Roy, P.K., Hakkarainen, M., Varma, I.K., and Albertsson, A.C. (2011). Environ Sci Technol 45, 4217-4227). The crucial first step of this chain of events, i.e. the oxidation of PE polymer, is usually carried out by abiotic factors such as light or temperature. Once the long polymeric molecules are broken down, a process that takes years of exposure to environmental factors in the wild, bacteria or fungi intervene and continue the job. This is the current paradigm driving the research field in biodegradation. Within this paradigm, several bacterial and fungal strains have been identified as capable of carrying on a certain extent of PE degradation. However, in most of the cases such degradation requires an aggressive pre-treatment of PE (heating, UV light, etc.) that accelerates the incorporation of oxygen into the polymer, making the abiotic oxidation the real bottleneck of the reaction (Wei, R., and Zimmermann, W. (2017). Microbial Biotechnology 10, 1308-1322; Restrepo-Florez, J.-M., Bassi, A. and Thompson, M. R. (2014). International Biodeterioration & Biodegradation 88, 83-90; Amobonye, A., Bhagwat, P., Singh, S., and Pillai, S. (2021). Sci Total Environ 759, 143536; Matjasic, T., et al. (2021). Science of the Total Environment752*;* Walsh, A.N., et al. (2021). Environ. Sci. Technol 55, 12383-12392). In the past decade a few microorganisms have been described as capable of acting on untreated PE17-23, although they require a significantly longer incubation time compared to experimental conditions with pre-oxidized PE.

The identification of enzymes from microorganisms capable of degrading untreated PE has proven a difficult task. In fact, no such enzyme has been identified yet, confirming the crucial limiting role of oxidation in the whole biodegradation chain. Reported enzymes capable of acting on polyolefin-derived plastics require a pre-treatment of the plastic material (Wei, R., and Zimmermann, W. (2017). Microbial Biotechnology 10, 1308-1322; Amobonye, A., Bhagwat, P., Singh, S., and Pillai, S. (2021). Sci Total Environ 759, 143536.). For example, two reported laccases, able to chemically modify PE, necessitate an abiotic pre-treatment or the addition of redox mediators such as 1-hydroxybenzotriazole.

This scenario confirms that the synthetic nature of the compound, together with the hydrophobicity and inaccessibility features, make plastic a difficult target for animal, fungal or microbial-derived enzymatic activities. Nonetheless, some lepidopteran and coleopteran insects revealed the unexpected capacity to degrade untreated PE and PS, for example that described in Yang, Y., Wang, J., and Xia, M. (2020). Sci Total Environ 708, 135233.

However, it is still needed to identify enzymes from microorganisms which are capable of biodegrading untreated PE, in particular of oxidating untreated PE polymers, avoiding thus the use of abiotic factors, such as light or temperature, long incubation times or aggressive pre-treatments of PE.

### DESCRIPTION OF THE INVENTION

The inventors have isolated an enzyme from the wax worm (*Galleria mellonella* larvae) saliva which has the unexpected capacity of oxidation and deterioration of untreated polyolefin-derived polymers, such as polyethylene (PE), at room temperature (RT), neutral pH and short incubation times. They achieved this by carrying out a proteomic analysis and a size exclusion chromatography (SEC) of the wax worm saliva, obtaining one enzyme identified as belonging to the hexamerin/prophenoloxidase family, re-named Cora (JHS), with accession number XP_026749149.1 (NCBI), SEQ ID NO: 1 (the enzyme of the invention). The capacity of this enzyme to oxidize/degrade polyolefin-derived polymers was tested on PE films (see Example and figures of the present patent application), showing a high degradation activity. This opens up a highway of possibilities to solve the plastic waste pollution issue.

This effect on PE degradation is achieved after only a few hours' exposure at room temperature and physiological conditions (neutral pH). Thus, no long incubations times or aggressive pre-treatments are needed. The enzyme provided in this invention can indeed overcome the bottleneck step in PE biodegradation, that is the initial oxidation step. Using Gas Chromatography-Mass Spectrometry (GC-MS) degradation products, such as small oxidized aliphatic chains, were identified, further confirming the breaking of the polymer in shorter molecules.

This enzyme is capable of producing such modifications on a PE film working at RT and in a very short time, embodying a promising alternative to the abiotic oxidation of plastic, the first and most difficult step in the degradation process. The identification of invertebrate enzymes capable of oxidizing PE in a few hours represents a totally new paradigm in the world of plastic degradation and more widely in the plastic waste management fields, and opens up a highway to design new formulae/routes for synthetic polymers production.

In view of the foregoing, in an aspect, the present invention relates to the use of:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1 (hereinafter, "the enzyme or the protein of the invention") or a functionally fragment thereof, or
- a host cell comprising a nucleotide sequence encoding said enzyme or a vector comprising a nucleotide sequence encoding said enzyme, (hereinafter, "the host cell of the invention"), or
- a composition comprising said enzyme or a functionally fragment thereof, or said host cell (hereinafter, "the composition of the invention"),
for biodegrading, or oxidating and/or depolymerizing, a polyolefin-derived polymer or a material comprising a polyolefin-derived polymer (hereinafter, "the use of the invention").

The enzyme of the invention is preferably isolated from the saliva of *Galleria mellonella* larvae, also known as wax worms (ww). It is the basic juvenile hormone-suppressible protein 1, re-named JHS (Cora), and comprises an amino acid sequence having a sequence identity of, at least, 60% with sequence SEQ ID NO: 1 (accession reference number NCBI: XP_026749149.1).

SEQ ID NO: 1 (JHS, Cora, the enzyme of the invention)

MVVTMRLVVAAVLLAAVAASVVHDELKNIVITKEPMKNMDMKSKEMCILKLMNHILQ
PTMYEDVREVAKAWVLEENEDKYMKMEAVKEFINTYKMGMLPRGEVFVHMDHKH
VEEAVKVFKLLYFANDFDVFLKTACWLRERINGGMFVYALTAAIFHRSDCSGIKIPA
PYEIYPYLFVDSNILHKAFMMKMSKAAMDPVMKNYYGIKVKDNSMVIIDWRKGLRH
TMSEFDRTSYFTEDIDLNTYLYYMHMSYPYWMNEDMYRVNKERRGEAMWYGYQ
QLQARLRLERLSHHMCDLKPLDLDGTLDEGYWPKILLHTGDEMPVRYNKMKLTNE
NNIKYRLLLEDNKRLIRDGIKKGHMAMHDGTTVSLKKPDDIENLCRIVLGGFVSKDD

HKGKSSIWRNLAKTMLSYGTYNMGKYTYIPTAADMYSTALRDPGMWKMLKLISEY
FIMFKEMLPKYTREELDFPGVKIEQVTTDKLVTFMDEYDVDITNAVYLDHDEMQKH
RSDMMYVARMHRLNHQPFKITIDVASDKAVECVVRVFLGPKLDCMGRFTSVNDKR
NDMVEIDSFLYKLETGKNTIVRDSLEMNNVIKERPWSRNNWAMDPSGGQKAQDN
WWYKSRIGFPHRLLLPMGSHGGMPYQMFVIVTPVRAGMSLPSIDMNTAKERKAC
RWTVCMDTMPLGFPFDRPIDETNFYTKNMKFHDVMVYTKDLAMSNMVKDVDMSE
MVMKRDDLTYLDKDMLVKRSYKSVMMMSGDDMTHM

Alternatively, the enzyme of the invention may be recombinantly produced in accordance with techniques well-known in the art. For example, using accepted techniques of chemical synthesis, the enzyme may be built up either from the N-terminus or, more typically, the C-terminus using either single amino acids or peptides containing two or more amino acid residues. Particular techniques for synthesizing enzymes include classical methods, classical chemical synthesis amino acid by amino acid and solid phase peptide synthesis in which the enzyme is built up attached to a resin, such as a Merrifield resin. In these synthetic procedures, groups on the amino acids will generally be in a protected form using standard protecting groups such as t-butoxycarbonyl. If necessary, these protecting groups are cleaved once the synthesis is complete. Chemistry synthesis methods, for example by peptide synthesis in solid phase, solution synthesis, a combination of methods of solid phase synthesis and solution or enzymatic synthesis, are known by the experts in the field. The enzyme of the present invention may also be produced through recombinant DNA procedures known in the art. Modifications may be introduced during or after the synthesis of the enzyme, for example to include a label attached for purification, a purification tag.

In another particular embodiment, the enzyme of the invention is codified and expressed from a nucleotide sequence which is comprised in an expression vector.

In the present invention, the term "identity" or "sequence identity" is understood to mean the degree of similarity between two nucleotide or amino acid sequences obtained by aligning the two sequences. Depending on the number of common residues between the aligned sequences, a different degree of identity, expressed as a percentage, will be obtained. The degree of identity between two amino acid sequences may be determined by conventional methods, for example, by standard sequence alignment algorithms known in the state of the art, such as, for example, BLAST. The BLAST programmes, for example, BLASTN, BLASTX, and TBLASTX, BLASTP and TBLASTN, are in the public domain at The National Center for Biotechonology Information (NCBI) website.

Persons skilled in the art understand that mutations in the nucleotide sequence of genes that lead to conservative amino acid substitutions at non-critical positions for the functionality of the protein are evolutionarily neutral mutations which do not affect its global structure or its functionality, giving rise to proteins which although comprise different amino acid sequence perform the same activity. These proteins are considered "functionally equivalent variants" of the sequence SEQ ID NO: 1 and fall within the scope of the present invention. Thus, the term "functionally equivalent variant", as used herein, means an enzyme which is derived from a native enzyme (SEQ ID NO: 1 in the present invention) by one or more deletions, insertions and/or substitutions of one or more amino acids at site(s) within its amino acid sequence and performs the same activity, i.e. it keeps the capacity of oxidizing untreated polyolefin-derived polymers, such as polyethylene (PE), at room temperature. Variants can be prepared using any mutagenesis procedure known in the art, such as site-directed mutagenesis, synthetic gene construction, semi-synthetic gene construction, random mutagenesis, shuffling, etc. All proteins having a sequence identity of at least 60% with the amino acid sequence of SEQ ID NO: 1 and capable of oxidizing untreated polyolefin-derived polymers are considered functionally equivalent variants in the context of the invention. An example of an assay to check if a given protein is a functionally equivalent variant of the enzyme of SEQ ID NO: 1 is disclosed in the examples of the present patent application. Functionally equivalent variants of the present invention have at least 20%, e.g., at least 40%, at least 50%, at least 60%, at least 70%, at least 80%, at least 90%, at least 95%, or at least 100% of the oxidizing activity of the native protein of SEQ ID NO: 1.

The present invention also encompasses functionally equivalent fragments of the enzyme of the invention. The term "functionally equivalent fragment" means a polypeptide/protein having one or more (e.g., several) amino acids absent from the amino and/or carboxy terminus of a native protein (in the present invention the sequence SEQ ID NO: 1) and shows the same activity/function that the native protein (in the present invention, the capacity of oxidizing untreated polyolefin-derived polymers at a room temperature). An example of an assay to check if a fragment of the protein of the invention is a functionally equivalent fragment of the SEQ ID NO: 1 is disclosed in the examples of the present patent application. Functionally equivalent fragments of the present invention have at least 20%, e.g., at least 40%, at least 50%, at least 60%, at least 70%, at least 80%, at least 90%, at least 95%, or at least 100% of the oxidizing activity of the native protein SEQ ID NO: 1.

In a preferred embodiment, the amino acid sequence of the enzyme of the invention comprises, or consists of, the sequence SEQ ID NO: 1.

The nucleotide sequence of the invention, encoding the enzyme of the invention, may further comprise other elements apart from the coding sequence, such as introns, non-coding sequences in the 3'and/or 5'ends, ribosome binding sites, or the like. The nucleotide sequence of the invention may also comprise sequences encoding for additional amino acids useful for increasing the enzyme stability or for allowing a more efficient enzyme purification.

The nucleotide sequence of the invention may be introduced into a vector or genetic construct, for example in a cloning or expression vector, in order to obtain a vector comprising said nucleotide sequence. Preferably, said vector is an appropriate vector for the expression and purification of the enzyme of the invention.

The term "genetic construct" or "vector", as used herein, refers to a nucleic acid molecule, monocatenary or bicatenary, that is isolated and modified to contain nucleic acid segments in a way that could not exist in nature. The term "nucleic acid construct" or "genetic construct" is synonymous to the term "expression cassette" when the nucleic acid construct contains the control sequences required for the expression of the nucleotide sequence of the invention. Therefore, the genetic construct of the invention may also comprise one or more control or regulatory sequences of gene expression, such as, but not limited to, promoter sequences, leader sequences, terminator sequences, polyadenylation sequences, signal sequences, regulators, enhancers, etc.

An "expression vector" is a linear or circular DNA molecule comprising at least the nucleotide sequence of the invention operationally linked to additional nucleotides provided for its expression. This vector that comprises the nucleic acid sequence of the invention can be introduced into a host cell in such a way that the vector is maintained as a chromosomal component or as an autoreplicating extracromosomal vector.

The expression vector referred to in the present invention may be any vector (e.g. plasmid or virus) that can be conveniently subjected to a recombinant DNA procedure and can produce the expression of the nucleotide sequence of the invention comprised in it. The choice of vector will normally depend on the compatibility of the vector with the host cell into which the vector is to be introduced. The expression vector can be, for example but not be limited to, a plasmid, a cosmid, a phage, a virus or viral vector, an artificial bacterial chromosome (BAC), an artificial yeast chromosome (YAC), or similar. Vectors within the context of the present invention can be linear or closed circular. Preferably, the expression vector of the invention is a baculovirus expression vector, more preferably a P2 baculovirus vector.

The "host cell", as used herein, includes any cell type which is susceptible to transformation, transfection, transduction, and the like with the nucleotide sequence or the expression vector of the invention as referred to above. The host cell may be prokaryote or eukaryote, preferably eukaryote, such as mammalian, insect, plant or fungal cell. In a preferred embodiment, the host cell is an insect cell, more preferably a sf9 cell.

The host cell of the invention comprises, therefore, at least the nucleic acid sequence of the invention, preferably by means of the vector of the invention, in a recombinant manner. The nucleotide of the invention or the vector of the invention is not naturally present in that cell, but has been intentionally introduced through genetic engineering procedures. The nucleotide sequence of the invention can encode the mature enzyme of the invention or a pre-protein consisting of a signal peptide linked to the mature enzyme that will have to be processed later in order to produce the mature enzyme.

The expression of the enzyme of the invention in the host cell of the invention may be induced by any procedure known in the art, such as the transformation of a suitable host cell with at least one nucleotide sequence of the invention or with the vector of the invention, and the culture of the transformed host cell under conditions that induce the expression of that nucleotide sequence in order to obtain the secreted and functional enzyme. Preferred conditions for inducing the expression of the nucleotide sequence are the incubation of the host cell at 27ºC for 48-72h.

The enzyme of the invention produced by the host cell host of the invention can be purified by a variety of procedures known in the art including, but not limited to, chromatography (e.g., ion exchange, affinity, hydrophobia, chromatofocus, and size exclusion), electrophoretic procedures (e.g. preparative isoelectric focusing), differential solubility (e.g. ammonium sulphate precipitation), SDS-PAGE, or extraction, in order to obtain a substantially pure enzyme.

In another preferred embodiment, the enzyme of the invention is recombinantly produced or it has been isolated from *Galleria mellonella,* particularly, from *G*. *mellonella* saliva.

Methods for isolating the enzyme of the invention from *Galleria mellonella* saliva are, without limitation chromatographic technics (such as size exclusion and ion exchange chromatgraphy) from wax worm saliva.

The composition of the invention comprises the enzyme of the invention or a functionally fragment thereof, or the host cell of the invention, and optionally other elements needed for the optimal activity of these or for their storage. These additional elements may be, for instance, buffers, other enzymes for example enzymes useful for biodegrading a polyolefin-derived polymer, antibiotics, or the like. Preferably, the composition of the invention further comprises a second enzyme comprising an amino acid sequence having a sequence identity of, at least, 60, 65, 70, 75, 80, 85, 90, 91, 92, 93, 94, 95, 96, 97, 98 or 99% with SEQ ID NO: 2, more preferably this additional protein comprises, or consist of, SEQ ID NO: 2.

SEQ ID NO: 2 (Cibeles, g181563)

MQTVLFLAALVSLAAAGYPQYHYDVETRKLDPSLLNIQTKVLSLLENWKQVNPDDE
YYKIGKEYNVEANMESYTNREVVTEFLSLYKAGFIPKNEVFSIFYENQALEVIALYRL
FYYAKDFETFYKTAAFARVWLNEGQFVYAFYLAVIHRADTRGIVLPAPYEIWPEYF
MNSDVLSKIYRIQMQKGLIIPEQGPYYGILSKDNAYYFYANYSGPLTYEDNENLLSY
FIEDIGWNSYYYYFHNRFPFWENGEQLIGPLKERRGEIYYYVYQKILARYYLERLAN
GLGEIPRFNWLDKYQTSYYPLLSSYQLPFAQRNDDYYLASGDNINDIQFIDTYEKTF
LQLLQKGQFKAYKQEVDLYNSKSINFVGNYWQSNADLYEKVPKRNYWRSYEATA
RRVLGAAPRSSINYENMNIPTALDFYQTSLRDPAFYQLYAKILDYINEYKEYLEPYS
QDVLHYVGVKINDVKVDKLVTYFEYFDWNATNAVYLSEQQLDTVSPSYIVRQPRLN
NKPFTVNIDIKSDVESEVVVKIFLGPKYDGNGLPISLEDNWINFIELDWFTHKLTSGQ
NKIARKSEEFFFFKDDSVSLFKIYELLSNGQVPSYMVDRYIYLPRRLILPRGTQRGF
PLQLFVVVYPYQAPVKEWESMRQYIVDNKPFGYPFDRPVTLPYYFNQPNMYFKDV
YVYQEGEQYPYYNSYWSQNQVSNH

In the present invention, "polyolefin-derived polymer" is a type of polymer with the general formula (CH₂CHR)ₙ where R is an alkyl group. They are usually derived from a small set of simple olefins (alkenes). Dominant in a commercial sense are polyethylene and polypropylene. More specialized polyolefins include polyisobutylene and polymethylpentene. They are all colorless or white oils or solids. The name of each polyolefin indicates the olefin from which it is prepared; for example, polyethylene is derived from ethylene, and polymethylpentene is derived from 4-methyl-1-pentene.

In another preferred embodiment, the polyolefin-derived polymer referred to in the present invention is polyethylene (PE).

"Polyethylene (PE)" or polythene (abbreviated PE; IUPAC name polyethene or poly(methylene)) is the most common plastic in use today. It is a polymer, primarily used for packaging (plastic bags, plastic films, geomembranes and containers including bottles, etc.). Many kinds of polyethylene are known, with most having the chemical formula (C₂H₄)ₙ. PE is usually a mixture of similar polymers of ethylene, with various values of n. It can be low-density or high-density: low-density polyethylene is extruded using high pressure (1000-5000 atm) and high temperature (520 kelvins), while high-density polyethylene is extruded using low pressure (6-7 atm) and low temperature (333-343 K). Polyethylene is usually thermoplastic, but it can be modified to become thermosetting instead, for example, in cross-linked polyethylene. All types of PE are encompassed within the scope of the present invention.

In an even more preferred embodiment, the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE). In a particular embodiment, the PE is LDPE, more preferably PE 4000 or PE 2000.

In another particular embodiment, the polyolefin-derived polymer or the material comprising a polyolefin-derived polymer is not pre-treated with abiotic factors (such as heating, UV light, etc,) previously to the biodegradation by the enzyme of the invention, the host cell of the invention or the composition of the invention, i.e. the enzyme of the invention is capable of biodegrading untreated polyolefin-derived polymer or untreated material comprising a polyolefin-derived polymer.

In another aspect, the present invention refers to a method, hereinafter "the method of the invention", for biodegrading, oxidating and/or depolymerizing a polyolefin-derived polymer, or a material comprising a polyolefin-derived polymer, wherein said method comprises contacting:
- the enzyme of the invention, or
- the host cell of the invention, or
- the composition of the invention,
with a polyolefin-derived polymer or a material comprising a polyolefin-derived polymer.

In a preferred embodiment of the method of the invention, said method is carried out at room temperature, preferably, at room temperature in an aqueous solution with a neutral pH.

"Room temperature" is from 15 °C to 30 °C, preferably 22°C.

"Neutral pH" is from pH 7 to pH 8.

In a preferred embodiment, the enzyme is used in the method of the invention in an amount between 2-10 µL, preferably 5 µL, and in a concentration between 1 and 5 µg/µL.

In another preferred embodiment of the method of the invention, the incubation time between the enzyme, the host cell or the composition of the invention, and the polyolefin-derived polymer is at least 60 to 120 min, preferably at least 90 min.

In an even more preferred embodiment of the method of the invention, the enzyme is used in an amount of 5 µL or 10 µL, in a concentration between 1 and 5 µg/µL, preferably 1.2 µg/µL, and is applied at least 8 times, preferably 24 times, on the polyolefin-derived polymer or a material comprising a polyolefin-derived polymer, 90 minutes each time.

In another preferred embodiment of the method of the invention, the enzyme of the invention comprises, or consists of, the sequence SEQ ID NO: 1. More preferably, the enzyme of the invention is isolated from *G. mellonella,* particularly, from *G. mellonella* saliva.

In another preferred embodiment of the method of the invention, the composition of the invention further comprises a second enzyme comprising an amino acid sequence having a sequence identity of, at least, 60, 65, 70, 75, 80, 85, 90, 91, 92, 93, 94, 95, 96, 97, 98 or 99% with SEQ ID NO: 2, more preferably comprising the SEQ ID NO: 2, even more preferably consisting of SEQ ID NO: 2.

In another preferred embodiment of the method of the invention, the polyolefin-derived polymer is polyethylene (PE).

In another preferred embodiment of the method of the invention, the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE). In a particular embodiment, the PE is LDPE, more preferably PE 4000 or PE 2000.

### DESCRIPTION OF THE DRAWINGS

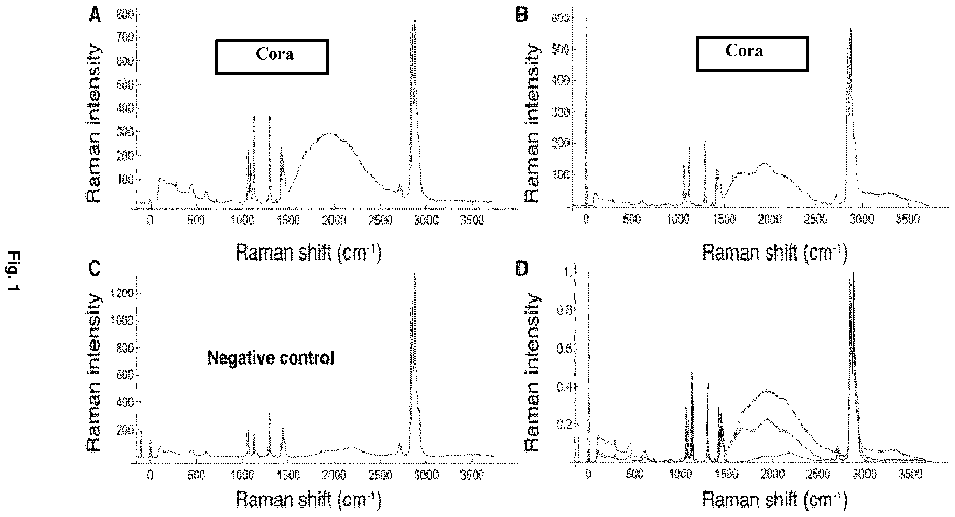

**Figure 1****. RAMAN analysis of PE film treated with purified recombinant Cora.** A, B. PE film treated with Cora. The peaks between 1500 and 2400 cm⁻¹ indicate PE deterioration. Oxidation is indicated between 1600 and 1800 cm⁻¹ (carbonyl group) and 3000-3500 cm⁻¹ (hydroxyl group) (evident in B). C. Control PE film. PE signature is characterized by the peaks at 1061, 1128, 1294, 1440, 2846 and 2880 cm⁻¹. D. Overlapping normalized profiles (A, B and C).

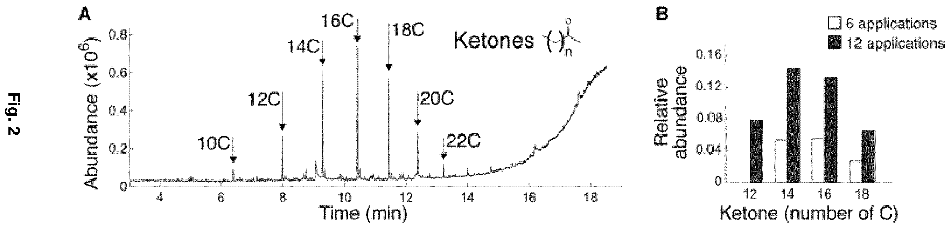

**Figure 2****. Identification via GC-MS of the degradation by-products of PE treated with Cora.** A. Chromatogram of the fragmentgram of the ion m/z 58 from methyl ketones of PE treated with the enzyme. The arrows indicated the peaks corresponding to 2-ketones with different number of carbons. B. Variation of PE degradation by-products via GC-MS after different applications. Increase of ketones formation as degradation products from 6 to 12 applications of Cora to PE, with increase of 2-ketones formation by doubling applications of the enzyme to PE.

### EXAMPLE. PE degradation experiments.

### I - RAMAN analysis

Recombinant protein of SEQ ID NO: 1 was applied as follows: 5 µl of protein (concentration between 1 and 5 µg/ml) were applied eight times on PE film 90 minutes each time.

RAMAN analyses were performed on (treated and control) PE films using Alpha300R - Alpha300A AFM Witec equipment with 5mW power, 50x (NA0.8) objective, integration time 1, accumulation 30, wavelength 532 nm. Results are shown in Fig. 1.

### II - Gas Chromatography Mass Spectrometry (GC-MS)

PE was exposed to 10 µl (1.2 µg/mL) of Cora (JHS) 24 times for 90 minutes. Prolonged treatment was performed (days 1 and 2), 4 applications per day of 10 µl (1.2 µg/mL) for 90 minutes each. As control, the same experiment was repeated using the protein buffer. Afterward, samples were centrifuged with an Eppendorf centrifuge 5810 R at 19083 g for 30 seconds and the subnatant was transferred to a new 1.5 ml Eppendorf tube. Samples and controls were extracted using a QuEChERS (quick, easy, cheap, effective, and safe) method with some modifications. Briefly, 50 µl of diphenyl phthalate (Internal Standard; IS) at a concentration of 1 mg/ml was added at each sample and extracted with 300 µl of dichloromethane (DCM) and 5 % (v/m) of NaCl. The tube was vortexed for 30 seconds and sonicated in a bath (50/60 Hz) for 15 min at room temperature, followed by centrifugation with an Eppendorf centrifuge 5810 R at 20 ºC and 19083 g for 10 min. Finally, DCM located as the subnatant was collected and placed in an insert before analysis. Silylation reaction with N, O-Bis(trimethylsilyl)trifluoroacetamide (BSTFA) was performed to determine the low-volatility polar compounds which show low detection sensibility. A fraction of 50 µl of each sample with 50 µl of BSTFA was incubated for 20 min at 60 ºC before the analysis.

Dichloromethane (DCM; CAS-No: 75-09-2) for gas chromatography-mass spectrometry (GC-MS) was SupraSolv grade purity and obtained from Sigma-Aldrich (Darmstadt, Germany). Sodium chloride (NaCl; ≥ 99.5 %; CAS-No: 7647-14-5) and ultrapure water from a Milli-Q system were supplied from Merck (Darmstadt, Germany).

Crystalline granular powder polyethylene (PE 4000; CAS-No: 9002-88-4) was supplied by Sigma-Aldrich (Saint Louis, USA).

Chromatographic analyses were performed with a gas chromatography-mass spectrometry system (GC-MS) 7980A-5975C from Agilent Technologies. Separation of the metabolites was performed on a DB-5^{th} Column coated with polyimide (30 m length, 0.25 mm inner diameter, and 0.1 µm film thickness; Agilent Technologies, USA) for proper separation of substances, and Helium (He) was utilized as a carrier gas. The analysis was performed using a split injector at 350 ºC and an injection volume of 1 µl. The ion source temperature was 230 ºC, the ºC mass spectral analysis was performed in scan mode, the quadrupole temperature of 150 ºC, and a fragmentation voltage of 70 eV. The oven program started at 60 ºC for 3 min, then 20 ºC/min to 350 ºC for 1 min. The total run time was 18.5 min and 19.5 min for derivatized samples. The resulting chromatograms were processed using the software MSD ChemStation E.01.00.237 from Agilent Technologies, Inc while for the identification NIST11 library was used.

The evaluation of the prolonged treatment was based on the relative abundance of each untargeted compound, which consists of the quotient of the area under the peak of each compound divided by the area under the peak of the IS.

Results are shown in Fig. 2.

### III - Protein buffer

The produced proteins were resuspended in 50mM Tris-Cl, 500 mM NaCl, 10% Glycerol, 0.5% Sodium Lauroyl Sarcosine, 2mM TCEP, 2mM glutathione, pH 7.5, and used for the degradation assay. The same buffer alone was used as negative control.

## Claims

1. Use of:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1, or
- a host cell comprising a nucleotide sequence encoding said enzyme, or
- a composition comprising said enzyme or said host cell,
for biodegrading a polyolefin-derived polymer or a material comprising a polyolefin-derived polymer.

2. Use according to claim 1, wherein the amino acid sequence comprises, or consists of, the sequence SEQ ID NO: 1.

3. Use according to claims 1 or 2, wherein the composition further comprises a second enzyme which comprises an amino acid sequence having a sequence identity of, at least, 60, 65, 70, 75, 80, 85, 90, 91, 92, 93, 94, 95, 96, 97, 98 or 99% with SEQ ID NO: 2.

4. Use according to any one of claims 1 to 3, wherein the polyolefin-derived polymer is polyethylene (PE).

5. Use according to claim 4, wherein the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE).

6. Use according to any one of claims 1 to 5, wherein the enzyme is isolated from *Galleria mellonella,* preferably, from *G. mellonella* saliva.

7. A method for biodegrading a polyolefin-derived polymer, or a material comprising a polyolefin-derived polymer, comprising contacting:
- an enzyme comprising an amino acid sequence having a sequence identity of, at least, 60, 65, 70, 75, 80, 85, 90, 91, 92, 93, 94, 95, 96, 97, 98 or 99% with SEQ ID NO: 1, or
- a host cell comprising a nucleotide sequence encoding said enzyme, or
- a composition comprising said enzyme or said host cell,
with a polyolefin-derived polymer or a material comprising a polyolefin-derived polymer.

8. Method according to claim 7, wherein the method is carried out at room temperature, preferably, at room temperature in an aqueous solution with a neutral pH.

9. Method according to claims 7 or 8, wherein the amino acid sequence comprises, or consists of, the sequence SEQ ID NO: 1.

10. Method according to any one of claims 7 to 9, wherein the composition further comprises a second enzyme which comprises an amino acid sequence having a sequence identity of, at least, 60, 65, 70, 75, 80, 85, 90, 91, 92, 93, 94, 95, 96, 97, 98 or 99% with SEQ ID NO: 2.

11. Method according to any one of claims 7 to 10, wherein the polyolefin-derived polymer is polyethylene (PE).

12. Method according to claim 11, wherein the PE is selected from the list consisting of: ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), and chlorinated polyethylene (CPE).

13. Method according to any one of claims 7 to 12, wherein the enzyme is isolated from *G. mellonella,* preferably, from *G. mellonella* saliva.
